# EUROPÄISCHE PATENTSCHRIFT

(11) **EP 2 215 469 B1**
(45) Veröffentlichungstag und Bekanntmachung des Hinweises auf die Patenterteilung: **15.01.2014**
(21) Anmeldenummer: 08849238.4
(22) Anmeldetag: 13.11.2008
(51) Int. Cl.: G01N 33/487

(54) **VERFAHREN ZUM ÜBERWACHEN DER NUTZUNG EINES VERBRAUCHSMATERIALS IN EINWEGAUSFÜHRUNG IN MEHREREN ANALYSEGERÄTEN**
METHOD FOR MONITORING THE USE OF CONSUMABLES OF A DISPOSABLE TYPE IN MULTIPLE ANALYSIS DEVICES
PROCÉDÉ DE SURVEILLANCE DE L'UTILISATION D'UN CONSOMMABLE À USAGE UNIQUE DANS PLUSIEURS APPAREILS D'ANALYSE

(30) Priorität: 13.11.2007 US 987442 P
(43) Veröffentlichungstag der Anmeldung: 11.08.2010
(73) Patentinhaber: Roche Diagnostics GmbH, 68305 Mannheim (DE); F. Hoffmann-La Roche AG, 4070 Basel (CH)
(72) Erfinder: RUETHER, Horst, 8047 Hart (AT); STEINBOECK, Wolf-Dietrich, 8044 Graz (AT); BARTEL, Arnold, 8051 Graz (AT); FELSBERGER, Robert, 8020 Graz (AT)
(74) Vertreter: Bittner, Thomas L.
(86) Internationale Anmeldenummer: PCT/EP2008/009615
(87) Internationale Veröffentlichungsnummer: WO 2009/062722

(56) Entgegenhaltungen:
- WO-A-03/082091
- WO-A-2005/040793
- WO-A-2006/069675

## Beschreibung

Die Erfindung bezieht sich auf ein Verfahren zum Überwachen der Nutzung eines Verbrauchsmaterials in Einwegausführung in mehreren Analysegeräten, die jeweils konfiguriert sind, eine oder mehrere Flüssigkeiten, insbesondere Körperflüssigkeiten, zu analysieren.

### Hintergrund der Erfindung

Medizinisch-diagnostische Analysegeräte sind für unterschiedliche Analysezwecke bekannt. Zu derartigen Analysegeräten gehören beispielsweise Analysegeräte zum Analysieren einer Körperflüssigkeit. Bei einem solchen Analysegerät kann es sich zum Beispiel um einen Blutgas-Analysator handeln. Blutgas-Analysatoren werden beispielsweise als Analysegeräte zum Bestimmen von diagnostisch relevanten Parametern bereitgestellt, nämlich der Blutgaswerte, der Elektrolytwerte, der Metabolitwerte, des Hämatokritwerts, von Hämoglobinparametern und / oder des Bilirubinwertes von Blutproben. Sie werden insbesondere zur dezentralen Bestimmung der vorgenannten Parameter in Vollblutproben eingesetzt. Aber auch Anwendungen in der Veterinärmedizin und die Verwendung von Serum-, Plasma-, Harn- und Dialysat-Proben sind möglich.

Zum Betrieb der Analysegeräte werden regelmäßig auch Verbrauchsmaterialien benutzt. Zu derartigen Verbrauchsmaterialien gehört beispielsweise eine sogenannte Sensorkassette, welche die für eine Analytbestimmung benötigten Sensoren enthält. Verbrauchsmaterialien sind weiterhin Flüssigkeitsbehälter oder Reagenzienpackungen, welche die zum Betrieb des Analysegerätes benötigten Funktionsfluide wie Kalibrierungslösungen, Waschlösungen, Referenzflüssigkeiten oder Reagenzienlösungen enthalten. Oft werden auch mehrere solcher Flüssigkeitsbehälter oder Reagenzienpackungen in sogenannten Fluidpacks zusammengefaßt. Auch kann ein Verbrauchsmaterial für eine automatisierte Qualitätskontrolle zur Verfügung gestellt werden, zum Beispiel in Form einer Kassette mit ampullierten Referenzlösungen. Zum Verbrauchsmaterial in einem solchen medizinisch-diagnostischen Analysegerät kann aber auch Papier, beispielsweise in Form einer Papierkassette oder -rolle, für einen internen Drucker gehören. Den vorgenannten Verbrauchsmaterialien ist gemeinsam, dass es sich um Verbrauchsmaterialien in sogenannter Einwegausführung handelt, also Einweg-Verbrauchsmaterialien, die beim Betrieb in einem oder auch mehreren Analysegeräten aufgebraucht und nicht regenerierbar oder wieder befüllbar sind. Insbesondere umfassen die Einweg-Verbrauchsmaterialien auch alle Einweg-Verbrauchsmaterialien, deren Inhalte nicht in einem einzigen Schritt, zum Beispiel bei einem analytischen Teststreifen, sondern im Betrieb nur teilweise und nacheinander verbraucht werden und mit welchen daher mehrere Messungen oder damit verbundene Aktionen wie Kalibrieren, Waschschritte oder dergleichen in einem oder auch nacheinander in mehreren Analysegeräten durchgeführt werden können. Mit solchen auch multi-use-Verbrauchsmaterialien genannten Verbrauchsmaterialien können unter Einbeziehung des jeweiligen Verbrauchsmaterials mehrere Aktionen wie Messzyklen, Kalibrierungszyklen, Qualitätskontrollzyklen, Reinigungszyklen und / oder Standby-Phasen nacheinander durchgeführt werden, wobei immer nur ein Teil des Inhalts des Verbrauchsmaterials verbraucht wird. Solche multi-use-Verbrauchsmaterialien sind häufig in Form von Kassetten oder Fluidpacks ausgebildet, welche für eine Vielzahl von Messungen oder damit verbundener Aktionen ausgelegt sind und als Einweg-Verbrauchsmaterialen beispielsweise nach vollständigem Gebrauch (beispielsweise kompletter Entleerung) oder nach Erreichen einer maximalen Nutzungsdauer als Gesamtheit entsorgt werden können.

Die Dokumente WO 03/082091 A2, WO 2005/040793 A1 und WO 2006/069675 A1 offenbaren jeweils Verfahren zum Überwachen der Nutzung eines solchen Verbrauchsmaterials in Einwegausführung, bei denen die Verwendung des Einweg-Verbrauchsmaterials beim Betrieb eines Analysegerätes erfasst und in einer dem Einweg-Verbrauchsmaterial zugeordneten Speichereinheit gespeichert wird.

Im Unterschied hierzu sind Verbrauchsmaterialien bekannt, die nach einer Nutzung einer Wiederaufbereitung zugeführt werden, um sie dann erneut zu verwenden. Hierbei handelt es sich dann um Verbrauchsmaterialien in Mehrwegausführung. Regelmäßig kann in diesem Zusammenhang eine mehrfache Wiederaufbereitung nach vorheriger Nutzung vorgesehen sein, so dass mehrere Nutzungszyklen nach jeweiliger vorheriger Wiederaufbereitung durchlaufen werden können. So ist in dem Dokument US 2005/0019213 A1 die Wiederaufbereitung von mikrofluidischen Vorrichtungen, sogenannten Mikrochips beschrieben. Es wird vorgeschlagen, die mikrofluidischen Vorrichtungen mit einem Datenspeichermodul zu versehen, um insbesondere die Anzahl von sogenannten Wasch- und Nutzungszyklen zu dokumentieren. Auf diese Weise kann beispielsweise festgestellt werden, ob eine mikrofluidische Vorrichtung eine vorgegebene maximale Anzahl von Nutzungszyklen oder vorgegebene maximale Anzahl von Wiederaufbereitungszyklen bereits erreicht oder sogar überschritten hat.

### Zusammenfassung der Erfindung

Aufgabe der Erfindung ist es, ein Verfahren zum Überwachen der Nutzung eines Verbrauchsmaterials in Einwegausführung in mehreren Analysegeräten, die jeweils konfiguriert sind, ein oder mehrere Flüssigkeiten, insbesondere Körperflüssigkeiten, zu analysieren, anzugeben, welches die Einhaltung von Nutzungsvorgaben in Verbindung mit dem Einweg-Verbrauchsmaterial ermöglicht. Darüber hinaus sollen die in Verbindung mit Analysegeräten geltenden Sicherheitsanforderungen eingehalten werden.

Diese Aufgabe wird erfindungsgemäß durch ein Verfahren zum Überwachen der Nutzung eines Verbrauchsmaterials in Einwegausführung in mehreren Analysegeräten nach dem unabhängigen Anspruch 1 gelöst. Vorteilhafte Ausgestaltungen des Verfahrens sind Gegenstand von abhängigen Unteransprüchen.

Die Erfindung umfasst ein Verfahren zum Überwachen der Nutzung eines Verbrauchsmaterials in Einwegausführung in mehreren Analysegeräten, die jeweils konfiguriert sind, ein oder mehrere Flüssigkeiten, insbesondere Körperflüssigkeiten, zu analysieren, wobei das Verfahren die folgenden Schritte umfasst:
- Bereitstellen eines Einweg-Verbrauchsmaterials in einem ersten Analysegerät,
- bestimmungsgemäßes Verwenden des Einweg-Verbrauchsmaterials beim Betrieb des ersten Analysegerätes,
- Erfassen von gebrauchsrelevanten Informationen für das Einweg-Verbrauchsmaterial, die das bestimmungsgemäße Verwenden des Einweg-Verbrauchsmaterials beim Betrieb des ersten Analysegerätes betreffen,
- Speichern von gebrauchsrelevanten Daten, die aus den gebrauchsrelevanten Informationen abgeleitet werden, in einer dem Einweg-Verbrauchsmaterial zugeordneten Speichereinheit,
- Entfernen des Einweg-Verbrauchsmaterials aus dem ersten Analysegerät,
- erneutes Bereitstellen des Einweg-Verbrauchsmaterials in einem zweiten Analysegerät,
- wenigstens teilweises Auslesen der gebrauchsrelevanten Daten aus der Speichereinheit in dem zweiten Analysegerät,
- Auswerten der ausgelesenen gebrauchsrelevanten Daten mittels einer Steuereinrichtung und
- Ableiten von Steuersignalen betreffend die Zulässigkeit und / oder die Art eines weiteren bestimmungsgemäßen Verwendens des Einweg-Verbrauchsmaterials beim Betrieb des zweiten Analysegerätes als Reaktion auf das Auswerten der ausgelesenen gebrauchsrelevanten Daten.

Mit der Erfindung ist die Möglichkeit geschaffen, einen bestimmungsgemäßen und sicheren Gebrauch eines Verbrauchsmaterials, welches während seines Lebenszyklus einmal oder mehrmals aus dem Analysegerät herausgenommen und wiedereingesetzt wird, zu ermöglichen. Ein erfindungsgemäßes Verbrauchsmaterial in Einwegausführung ist nach seiner Herstellung und seinem vollständigen oder auch teilweisen Verbrauch nicht wieder regenerierbar, insbesondere nicht wieder befüllbar. Der Gebrauch kann in mehreren Analysegeräten stattfinden, welche für eine Vielzahl von Analysezyklen ausgelegt sind.

Die Einweg-Verbrauchsmaterialien sind als multi-use-Verbrauchsmaterialien ausgelegt, d.h. unter Einbeziehung des jeweiligen Verbrauchsmaterials können mehrere Aktionen wie Messzyklen, Kalibrierungszyklen, Qualitätskontrollzyklen, Reinigungszyklen und / oder Standby-Phasen nacheinander durchgeführt werden, wobei regelmäßig nur ein Teil des Inhalts des Verbrauchsmaterials verbraucht oder genutzt wird. Bei den Verbrauchsmaterialien handelt sich also um multi-use-Verbrauchsmaterialien in Einwegausführung, welche einerseits eine Vielzahl von Messungen und / oder damit zusammenhängender Aktionen ermöglichen, andererseits aber nach bestimmungsgemäßem Ge- oder Verbrauch in geeigneter Weise, vorzugsweise als Gesamtheit, entsorgt werden.

Der nacheinander erfolgende Gebrauch eines solchen multi-use-Einweg-Verbrauchsmaterials in verschiedenen oder auch demselben Analysegerät ist insbesondere dann sinnvoll, wenn das Einweg-Verbrauchsmaterials relativ selten verwendet wird und beispielsweise zusätzlich eine begrenzte Einsatzdauer hat. Dies kann beispielsweise bei Qualitätskontrollmedien der Fall sein, welche nur in bestimmten, relativ lange auseinander liegenden Zeitabständen oder nach einer bestimmten Anzahl von Analytbestimmungen eingesetzt werden. Hier kann durch eine kombinierte Nutzung solcher multi-use-Einweg-Verbrauchsmaterialien für mehrere Analysesysteme die Nutzung dieser Einweg-Verbrauchsmaterialien optimiert werden, indem beispielsweise die maximal mögliche Anzahl von Qualitätskontrollmessungen innerhalb des durch die Haltbarkeitsdauer vorgegebenen Zeitraums durchgeführt werden kann, was zu erhöhter Wirtschaftlichkeit und verringertem Abfallanfall führt. Andere Beispiele für sinnvolle Einsatzmöglichkeiten des Verfahrens sind zum Beispiel teure Sensoren für selten durchgeführte Analytbestimmungen, welche so nur einmal oder in wenigen Ausführungen vorrätig sein müssen, da sie je nach Einsatzzweck und Notwendigkeit zwischen verschiedenen Analysegeräten ausgetauscht werden können, oder auch Sensoren, welche zum Beispiel auf erhöhte Temperaturen während des Einsatzes im Analysegerät mit einer verringerten Lebensdauer reagieren und daher nach einer Messung aus dem Analysegerät entnommen, bei verträglicheren Umweltbedingungen gelagert und zur nachfolgenden Messung wieder in dasselbe oder auch ein anderes Analysegerät eingesetzt werden können.

Mit Hilfe der Erfassung und der Speicherung der gebrauchsrelevanten Daten kann für das jeweilige Einweg-Verbrauchsmaterial verfolgt werden, unter welchen Bedingungen der bisherige Gebrauch des Verbrauchsmaterials stattfindet. Die gebrauchsrelevanten Daten können verschiedenste Aspekte des Verwendens des Einweg-Verbrauchsmaterials im Betrieb des einen oder der mehreren Analysegeräte betreffen. Wenn das Einweg-Verbrauchsmaterial nach dem vorherigen Entfernen aus einem ersten Analysegerät in dieses oder in ein zweites Analysegerät wieder eingesetzt wird, werden diese in der dem Einweg-Verbrauchsmaterial zugeordneten Speichereinheit abgelegten gebrauchsrelevanten Daten ausgewertet, um festzustellen, ob der vom Nutzer nun beabsichtigte weitere Gebrauch überhaupt und in korrekter Weise stattfinden kann. Es werden hierzu entsprechende Steuersignale erzeugt, die dann für weitergehende Aktionen ausgewertet werden können. Auf diese Weise wird eine Entscheidung über die Zulässigkeit und / oder die Art eines weiteren bestimmungsgemäßen Gebrauchs getroffen. Die Steuersignale stellen insofern elektronische Informationen dar, die die Entscheidung oder die Entscheidungen bezüglich der Zulässigkeit und / oder der Art des weiteren bestimmungsgemäßen Gebrauchs anzeigen. Hieraus können dann der Entscheidung entsprechend beliebige weitere Aktionen abgeleitet werden, zum Beispiel eine entsprechende Benutzerinformation und / oder Steuerung des Analysegerätes.

Es kann vorgesehen sein, die Speichereinheit, in welche die gebrauchsrelevanten Daten gespeichert werden, direkt und fest, sei es lösbar oder nicht lösbar, auf dem Einweg-Verbrauchsmaterial anzubringen. Beispielsweise kann ein elektronisches Speichermodul verwendet werden, bei dem es sich um einen Speicherchip oder einen RFID-Chip handeln kann. Bevorzugt verfügt die Speichereinheit über einen nichtflüchtigen Speicher.

Das Ableiten der Steuersignale kann in einer bevorzugten Ausgestaltung des Verfahrens den folgenden Schritt umfassen: Erzeugen von Steuersignalen durch eine Steuereinrichtung als Reaktion auf das Auswerten der ausgelesenen gebrauchsrelevanten Daten, wobei die Steuersignale die Zulässigkeit eines weiteren bestimmungsgemäßen Verwendens des Einweg-Verbrauchsmaterials anzeigen, wenn beim Auswerten der ausgelesenen gebrauchsrelevanten Daten festgestellt wird, dass das weitere bestimmungsgemäße Verwenden des Einweg-Verbrauchsmaterials beim Betrieb des zweiten Analysegerätes zugelassen ist, und wobei die Steuersignale die Unzulässigkeit des weiteren bestimmungsgemäßen Verwendens des Einweg-Verbrauchsmaterials anzeigen, wenn beim Auswerten der ausgelesenen gebrauchsrelevanten Daten festgestellt wird, dass das weitere bestimmungsgemäße Verwenden des Einweg-Verbrauchsmaterials beim Betrieb des ersten oder des zweiten Analysegerätes unzulässig ist.

Eine bevorzugte Weiterbildung der Erfindung sieht vor, dass weiterhin die folgenden Schritte vorgesehen sind, wenn die Steuersignale die Zulässigkeit des weiteren bestimmungsgemäßen Verwendens des Einweg-Verbrauchsmaterials anzeigen: weiteres bestimmungsgemäßes Verwenden des Einweg-Verbrauchsmaterials beim Betrieb des zweiten Analysegerätes, Erfassen von weiteren gebrauchsrelevanten Informationen, die das weitere bestimmungsgemäße Verwenden des Einweg-Verbrauchsmaterials beim Betrieb des des zweiten Analysegerätes betreffen, und Speichern von weiteren gebrauchsrelevanten Daten, die aus den weiteren verbrauchsmaterialrelevanten Informationen abgeleitet werden, in der dem Einweg-Verbrauchsmaterial zugeordneten Speichereinheit. Wenn also die Steuersignale die Zulässigkeit des weiteren bestimmungsgemäßen Verwendens des Einweg-Verbrauchsmaterials anzeigen, erfolgt eine weitere Nutzung in identischer oder aufgrund der bisherigen Nutzung abgewandelter Art und Weise, die ihrerseits dann dahingehend überwacht wird, zum Beispiel mit Hilfe einer im Analysegerät befindlichen Überwachungseinrichtung, dass weitere gebrauchsrelevante Informationen erfasst und hiervon abgeleitete Daten gespeichert werden. Eine aufgrund der bisherigen Nutzung des Verbrauchsmaterials abgewandelte weitere Nutzung kann beispielsweise bei einer Sensorkassette als Einweg-Verbrauchsmaterial dahingehend erfolgen, dass nach einmaligem Durchlaufen eines Konditionierungsprozesses, zum Beispiel eine sogenannte wetup-Phase bei trocken gelagerten elektrochemischen Sensoren, bei erstmaligem Gebrauch der Sensorkassette dieser Konditionierungsprozess beim erneuten späteren Einsetzen der gleichen Sensorkassette in das gleiche oder in ein anderes Analysegerät nicht mehr zwangsweise durchlaufen werden muss. Die weiteren gebrauchsrelevanten Daten können in Ergänzung zu den bereits in der Speichereinheit existierenden Daten gespeichert werden, so dass eine komplette Historie aller dieser gebrauchsrelevanten Daten auf dem Speicherelement vorliegt. Aber auch ein teilweiser Ersatz der ursprünglich gespeicherten Daten durch die weiteren gebrauchsrelevanten Daten kann vorgesehen sein, indem bestehende gebrauchsrelevante Daten zumindest teilweise aktualisiert und überschrieben werden können.

Bei einer bevorzugten Ausführungsform der Erfindung ist vorgesehen, dass aus den ausgelesenen gebrauchsrelevanten Daten die Art des weiteren bestimmungsgemäßen Verwendens des Einweg-Verbrauchsmaterials beeinflussende Daten abgeleitet werden. Ausgehend von den abgeleiteten Daten wird dann das weitere bestimmungsgemäße Verwenden des Einweg-Verbrauchsmaterials in dem zweiten Analysegerät gesteuert. Beispielsweise können anhand der gespeicherten gebrauchsrelevanten Daten Kenndaten von Referenzmaterialien, beispielsweise Sollkonzentrationen von in den Referenzmaterialien enthalten Substanzen, welche beim weiteren bestimmungsgemäßen Verwenden des Einweg-Verbrauchsmaterials in dem Analysegerät herangezogen werden, nachgestellt werden. Wenn in einer solchen Ausführungsform beispielsweise aus den ausgelesenen gebrauchsrelevanten Daten abgeleitet wird, dass das Einweg-Verbrauchsmaterial (in diesem Falle die Referenzmaterialien) eine bestimmte Temperaturbelastung bei einer früheren bestimmungsgemäßen Nutzung erfahren hat, können zum Beispiel temperaturabhängige Kenngrößen des Referenzmaterials für das weitere bestimmungemäße Verwenden des Einweg-Verbrauchsmaterials nachgestellt werden. So kann eine von dem Einweg-Verbrauchsmaterial früher erfahrene Temperaturbelastung, die zum Beispiel durch eine bestimmte Temperatur über eine festgestellte Zeitdauer charakterisiert wird, zu einer prozentmäßigen Nachstellung einer oder mehrerer temperaturabhängiger Kenngrößen des Referenzmaterials führen. Dieses kann beispielsweise in Verbindung mit organischen Referenzsubstanzen wie Glucose oder Urea (Harnstoff) zur Anwendung kommen, bei denen ein beschleunigter Abbau bei höheren Temperaturen und somit entsprechende Änderungen der Sollkonzentrationen dieser Referenzsubstanzen auftreten.

Bei einer weiteren zweckmäßigen Ausgestaltung der Erfindung kann vorgesehen sein, dass eine auf die Unzulässigkeit des weiteren bestimmungsgemäßen Verwendens des Einweg-Verbrauchsmaterials hinweisende Benutzerinformation erzeugt wird, wenn die Steuersignale die Unzulässigkeit des weiteren bestimmungsgemäßen Verwendens des Einweg-Verbrauchsmaterials anzeigen. Die Benutzerinformation kann optional eine akustische und / oder eine optische Signalisierung für den Benutzer umfassen. Verfügt das Analysegerät über ein Display, kann dort zum Beispiel eine entsprechende Textnachricht für den Benutzer dargestellt werden. Eine solche Anzeige weist den Benutzer beispielsweise darauf hin, dass ein weiterer Betrieb des Analysegerätes mit diesem speziellen Einweg-Verbrauchsmaterial zu unterbleiben hat.

Eine vorteilhafte Ausführungsform der Erfindung sieht vor, dass das weitere bestimmungsgemäße Verwenden des Einweg-Verbrauchsmaterials in dem zweiten Analysegerät unterbunden wird, wenn die Steuersignale die Unzulässigkeit des weiteren bestimmungsgemäßen Verwendens des Einweg-Verbrauchsmaterials anzeigen. Bei dieser Ausgestaltung werden die Steuersignale der Unzulässigkeit dahingehend ausgewertet, dass die weitere Verwendung des Einweg-Verbrauchsmaterials verhindert wird. Mittels einer geeigneten Signalisierung kann in einer Ausgestaltung der Benutzer hierüber zusätzlich informiert werden, beispielsweise mit Hilfe eines akustischen und / oder eines optischen Signals.

Bevorzugt sieht eine Fortbildung der Erfindung vor, dass die erfassten gebrauchsrelevanten Informationen und die erfassten weiteren gebrauchsrelevanten Informationen ein oder mehrere Informationsaspekte ausgewählt aus der folgenden Gruppe von Informationsaspekten umfassen:
- ein oder mehrere Nutzungsbedingungen beim bestimmungsgemäßen und / oder beim weiteren bestimmungsgemäßen Verwenden des Einweg-Verbrauchsmaterials wie chemische Nutzungsparameter und / oder physikalische Nutzungsparameter,
- eine Nutzungsdauer beim bestimmungsgemäßen und / oder beim weiteren bestimmungsgemäßen Verwenden des Einweg-Verbrauchsmaterials oder die Dauer des Aufenthalts des Einweg-Verbrauchsmaterials außerhalb eines Analysegeräts nach Entnahme aus dem ersten Analysegerät,
- einen Einsatzzeitpunkt des Einweg-Verbrauchsmaterials in das erste oder das zweite Analysegerät,
- eine Nichtnutzungsdauer des Einweg-Verbrauchsmaterials nach Entnahme aus dem ersten Analysegerät,
- unter Einbeziehung des mit dem Einweg-Verbrauchsmaterial durchgeführte Aktionen wie Messzyklen, Kalibrierungszyklen, Qualitätskontrollzyklen, Reinigungszyklen und / oder Standby-Phasen, und
- Verbrauchsmenge und / oder Verbrauchsumfang des Einweg-Verbrauchsmaterials beim bestimmungsgemäßen und / oder beim weiteren bestimmungsgemäßen Verwenden, zum Beispiel die Anzahl verbrauchter Ampullen oder die Anzahl und / oder Menge verbrauchter Reagenzien.

Die Nichtnutzungsdauer des Einweg-Verbrauchsmaterials nach der Entnahme aus dem Analysegerät betrifft den Zeitraum außerhalb eines Analysegerätes. Soweit als Informationsaspekt die Verbrauchsmenge und / oder der Verbrauchsumfang des Einweg-Verbrauchsmaterials betroffen sind, kann zum Beispiel eine Entnahme von Teilmengen des Inhalts des Einweg-Verbrauchsmaterials erfasst werden.

Zu den geeigneten Nutzungsparametern gehören allgemein alle Parameter, welche Informationen über Bedingungen oder Art und Weise des Verwendens des jeweiligen Einweg-Verbrauchsmaterials liefern können, welche sich auf das weitere bestimmungsgemäße Verwenden des jeweiligen Verbrauchsmaterials in diesem oder auch einem anderen Analysegerät auswirken können. Solche Nutzungsparameter können bevorzugt physikalische oder chemische Messgrößen und daraus abgeleitete Werte sein.

Zu den physikalischen Nutzungsparametern gehört beispielsweise eine Information über die Temperatur, welcher das Einweg-Verbrauchsmaterial beim Betrieb des Analysegerätes ausgesetzt ist. Ein anderer möglicher physikalischer Nutzungsparameter ist beispielsweise die Luftfeuchte, welche sich nachteilig auf bestimmte Eigenschaften wie die Haltbarkeit des Einweg-Verbrauchsmaterials auswirken kann. Auch Informationen über temperaturabhängige Stoffkonzentrationen sowie zeitabhängige Konzentrationsänderungen können für das Einweg-Verbrauchsmaterial erfasst und gespeichert werden. Auf diese Weise ist es beispielsweise ermöglicht, eine Temperaturbelastung für das Einweg-Verbrauchsmaterial beim Verwenden zu dokumentieren. Hierdurch können beispielsweise Zerfallsraten von Analyten in Referenzmaterialien berechnet werden und damit Kenndaten von Referenzmaterialien nachgestellt werden. Weiters können daraus gegebenenfalls Rückschlüsse gezogen werden, ob das Verbrauchsmaterial vielleicht wegen zu starker Temperaturbelastung beschädigt und damit nicht mehr verwendbar ist.

Ein chemischer Nutzungsparameter ist beispielsweise ein gebrauchsspezifischer Analytgehalt einer im Verbrauchsmaterial enthaltenen Flüssigkeit - beispielsweise der Sauerstoffpartialdruck oder die Sauerstoffkonzentration einer bestimmten Funktionsflüssigkeit -, welche mit Hilfe eines von der Überwachungseinrichtung verwendeten geeigneten Sensors bestimmt werden kann. Hierdurch kann eine qualitative und / oder quantitative Änderung von weiteren Inhaltsstoffen des Einweg-Verbrauchsmaterials - beispielsweise ein hiermit in Verbindung stehender, beispielsweise oxidativer, Abbau organischer Inhaltsstoffe - ermittelt werden, um optional hiervon ausgehend Referenzwerte beim bestimmungsgemäßen Verwenden des Einweg-Verbrauchsmaterials in dem Analysegerät für den weiteren Gebrauchsprozess ein- oder nachzustellen. In ähnlicher Weise kann dieses Nachstellen im Zusammenhang mit Sensoren vorgesehen sein, die andere chemische oder physikalische Parameter des Einweg-Verbrauchsmaterials erfassen. Im Fall einer Sensorkassette umfassend einen oder mehrere Biosensoren kann beispielsweise eine Aktivitätsabnahme der in den Biosensoren enthaltenen Enzyme in Folge sowohl der Anzahl der bereits damit durchgeführten Messungen als auch der jeweiligen Analytkonzentrationen der vermessenen Flüssigkeitsproben erfasst werden.

Bei einer vorteilhaften Ausgestaltung der Erfindung kann vorgesehen sein, dass beim Erfassen von gebrauchsrelevanten und / oder beim Erfassen von weiteren gebrauchsrelevanten Informationen Teilinformationen in zeitlichen Abständen erfasst werden. Auf diese Weise ist es beispielsweise ermöglicht, die gebrauchsrelevanten Informationen regelmäßig oder stichprobenartig während des Gebrauchs des Einweg-Verbrauchsmaterials zu erfassen.

Eine Weiterbildung der Erfindung kann vorsehen, dass das Erfassen von gebrauchsrelevanten Informationen und / oder das Erfassen von weiteren gebrauchsrelevanten Informationen jeweils vor, während und / oder nach dem bestimmungsgemäßen / dem weiteren bestimmungsgemäßen Verwenden des Einweg-Verbrauchsmaterials erfolgt.

Eine bevorzugte Weiterbildung der Erfindung sieht vor, dass beim Speichern von gebrauchsrelevanten Daten und / oder Speichern von weiteren gebrauchsrelevanten Daten Teildaten in zeitlichen Abständen gespeichert werden.

Eine vorteilhafte Ausführung der Erfindung kann vorsehen, dass die gebrauchsrelevanten Daten vor dem Entfernen des Einweg-Verbrauchsmaterials aus dem ersten Analysegerät und / oder die weiteren gebrauchsrelevanten Daten vor dem Entfernen des Einweg-Verbrauchsmaterials aus dem ersten oder dem zweiten Analysegerät in der zugeordneten Speichereinheit gespeichert werden. Hierdurch ist gewährleistet, dass zumindest zu diesem Zeitpunkt alle für eine eventuelle weitere Nutzung des Einweg-Verbrauchsmaterials notwendigen gebrauchsrelevanten Informationen auf dem diesem Einweg-Verbrauchsmaterial zugeordneten Speicherelement gespeichert sind und so zusammen mit dem Einweg-Verbrauchsmaterial bei weiteren Einsatz dessen in einem weiteren Analysegerät an dieses weitergegeben werden können.

Bei einer zweckmäßigen Ausgestaltung der Erfindung kann vorgesehen sein, dass nach dem Bereitstellen des Einweg-Verbrauchsmaterials in dem ersten oder dem zweiten Analysegerät weiterhin vom Ge- oder Verbrauch des Einweg-Verbrauchsmaterials unabhängige Daten ausgelesen werden. Vom Ge- oder Verbrauch des Einweg-Verbrauchsmaterials unabhängige Daten können beispielsweise ein Herstellungsdatum, ein Ablaufdatum oder chargenspezifische Informationen umfassen. Auch kann vorgesehen sein, dass die vom Verbrauch des Einweg-Verbrauchsmaterials unabhängigen Daten Informationen darüber umfassen, mit welchen Gerätetypen von Analysegeräten das jeweilige Einweg-Verbrauchsmaterial genutzt werden darf. Beim Einsetzen des Einweg-Verbrauchsmaterials in das Analysegerät können die vom Gebrauch bzw. Verbrauch unabhängigen Daten ganz oder teilweise ausgewertet werden, um in Abhängigkeit davon, eventuell auch in Kombination mit gebrauchsrelevanten Daten, zum Beispiel den Gebrauch in dem Analysegerät freizugeben oder zu sperren.

Bei einer vorteilhaften Ausgestaltung der Erfindung kann vorgesehen sein, dass das Erfassen der gebrauchsrelevanten Informationen und / oder der weiteren gebrauchsrelevanten Informationen für das Einweg-Verbrauchsmaterial mit Hilfe einer Überwachungseinrichtung des ersten / zweiten Analysegerätes ausgeführt wird. Die Überwachungseinrichtung des jeweiligen Analysegerätes ist konfiguriert, die gebrauchsrelevanten Informationen und / oder die weiteren gebrauchsrelevanten Informationen für das Einweg-Verbrauchsmaterial zu erfassen, indem zum Beispiel eine den zu erfassenden Informationen angepasste Sensorik vorgesehen ist.

Eine Weiterbildung der Erfindung kann vorsehen, dass das Speichern der gebrauchsrelevanten Daten und / oder das Speichern der weiteren gebrauchsrelevanten Daten in der dem Einweg-Verbrauchsmaterial zugeordneten Speichereinheit mit Hilfe einer Schreibeinrichtung des ersten / zweiten Analysegerätes ausgeführt wird. Die Schreibeinrichtung des jeweiligen Analysegerätes ist konfiguriert, mittels Datenkommunikation, zum Beispiel mit Hilfe eines drahtlosen Datenaustausches, die gebrauchsrelevanten Informationen und / oder die weiteren gebrauchsrelevanten Informationen in die zugeordnete Speichereinheit zu schreiben.

Eine bevorzugte Weiterbildung der Erfindung sieht vor, dass das wenigstens teilweise Auslesen der gebrauchsrelevanten Daten aus der Speichereinheit mit Hilfe einer Leseeinrichtung des ersten / zweiten Analysegerätes ausgeführt wird. Die Leseeinrichtung des jeweiligen Analysegerätes ist konfiguriert, mittels Datenkommunikation, zum Beispiel mit Hilfe eines drahtlosen Datenaustausches, die gebrauchsrelevanten Informationen aus der zugeordneten Speichereinheit auszulesen.

### Beschreibung bevorzugter Ausführungsbeispiele der Erfindung

Die Erfindung wird im Folgenden anhand von bevorzugten Ausführungsbeispielen unter Bezugnahme auf eine Figur näher erläutert.

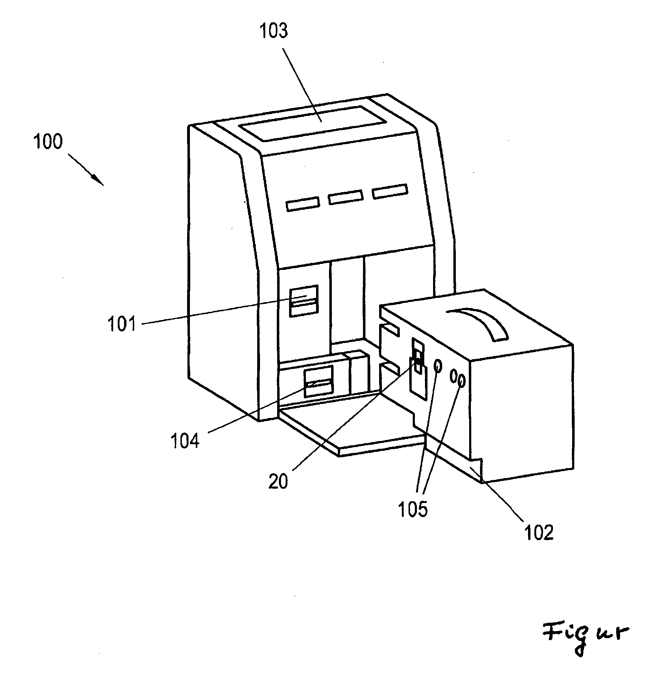

Die einzige Figur zeigt schematisch ein Analysegerät 100, bei dem es sich um ein Analysegerät zum Bestimmen einer Körperflüssigkeit handelt, beispielsweise einen Blutgasanalysator. Dieses Analysegerät 100 ist als "beinahe wartungsfrei" ausgelegt, so dass alle zum laufenden Betrieb benötigten Verbrauchsmaterialien als Einweg-Verbrauchsmaterialien in Form von Kassetten und / oder Modulen, die auch als "Consumables" bezeichnet werden, vorliegen und daher auch von (technisch) ungeschultem Personal getauscht werden können. Die verwendeten Einsatz- und Verbrauchsmaterialien sind in diesem Ausführungsbeispiel in folgenden Einweg-Verbrauchsmaterialien zusammengefasst:
- Eine Sensorkassette 101, welche zumindest einen Teil, vorzugsweise alle der für die Analytbestimmung benötigten Sensoren enthält.
- Ein Fluidpack 102, das Flüssigkeits- und Abfallbehälter enthält, welche die zum Betrieb des Analysegerätes 100 benötigten Betriebs- oder Funktionsflüssigkeiten, wie beispielsweise Kalibrations- oder Referenzflüssigkeiten, Wasch- und Reinigungsflüssigkeiten oder auch Reagenzienflüssigkeiten, enthält. Optional können im Fluidpack 102 auch weitere Elemente oder Funktionalitäten wie das gesamte Fluidiksystem oder Teile hiervon wie die Probeneingabevorrichtung oder auch weitere sensorische Komponenten enthalten sein.
- Eine Druckerpapierkassette 103 für einen internen Drucker.
- Optional eine Qualitätskontrollkassette 104 mit Referenzlösungen in Ampullenform zur Durchführungen einer automatisierten Qualitätskontrolle, welche das Personal durch einfache intuitive Handgriffe selbst tauschen kann.

Die hier geschilderte Untergliederung der Einweg-Verbrauchsmaterialen ist nur exemplarisch. Es kann auch vorgesehen sein, dass (Teil-)Funktionalitäten oder (Teil-)Elemente mehrerer Einweg-Verbrauchsmaterialien zusammengefasst werden, so dass beispielsweise weniger Einweg-Verbrauchsmaterialien oder sogar nur ein Einweg-Verbrauchsmaterial benötigt werden. Andererseits ist es auch denkbar, dass (Teil-)Funktionalitäten oder (Teil-)Elemente einzelner Einweg-Verbrauchsmaterialien auf mehrere verteilt werden , zum Beispiel auf mehrere Sensorkassetten oder -module.

Die Einweg-Verbrauchsmaterialien werden untereinander oder mit dem Analysegerät 100 mittels aufeinander abgestimmter Schnittstellen gekoppelt, zum Beispiel in Form fluidischer Andocknippel 105 oder auch elektrischer Kontakte (nicht dargestellt). Das mechanische Verbinden der Einweg-Verbrauchsmaterialien mit den respektiven Gegenteilen kann mit Hilfe eines einfachen manuellen Bewegungsablaufes direkt durch den Benutzer erfolgen, oder mittels im Gerät befindliche Antriebe, die die Ankopplung automatisch durchrühren, nachdem der Benutzer die Kassette nur in "Position" gebracht hat.

Beispielsweise kann eine Sensorkassette 101 als Einweg-Verbrauchsmaterial in eine zugeordnete Öffnung des Analysegeräts 100 eingesetzt und hieraus entfernt werden. In ähnlicher Weise kann das Einsetzen / Herausnehmen des Fluidpacks 102 in eine zugeordnete Aufnahme des Analysegeräts 100 erfolgen.

Der Flüssigkeitsbehälter 102 enthält die für den Betrieb des Analysegerätes 100 notwendigen Funktionsflüssigkeiten, zum Beispiel in Form von Ampullen oder anderer Behälter, welche Teilvolumen der darin enthaltenen Flüssigkeiten an das Analysegerät 100 oder andere Einweg-Verbrauchsmaterialien abgeben können. Beim Einsetzen des Flüssigkeitsbehälters 102 erfolgt das Ankoppeln an das Analysegerät 100, zum Beispiel über fluidische Andocknippel 105. Bevorzugt erfolgt nach dem Einsetzen der verschiedenen Einweg-Verbrauchsmaterialien eine mechanische Arretierung in dem Analysegerät 100, um ein unbeabsichtigtes Herausfallen oder Entnehmen zu vermeiden.

Wenn nun eines der vorangehend genannten Einweg-Verbrauchsmaterialien in das Analysegerät 100 eingesetzt wird, erfolgt ein Auslesen eines elektronischen Speichermoduls 20, welches an dem jeweiligen Einweg-Verbrauchsmaterial angeordnet ist, wie im dargestellten Falle auf dem Fluidpack 102. Bei dem elektronischen Speichermodul 20 handelt es sich beispielsweise um einen Speicherchip oder einen RFID-Chip. Zu diesem Zweck verfügt das Analysegerät 100 über zugeordnete Schreib- / Lesemittel (nicht dargestellt), die konfiguriert sind, elektronische Daten in das elektronische Speichermodul 20 einzuschreiben und hieraus zu lesen. Die ausgelesenen Daten werden in einer Steuereinrichtung, die zum Beispiel einen Mikroprozessor mit einer entsprechenden Software-Programmierung umfasst, ausgewertet, um hiervon abgeleitete Steuersignale zu erzeugen, die anzeigen, ob ein bestimmungsgemäßer Gebrauch des eingesetzten Einweg-Verbrauchsmaterials in dem Analysegerät 100 zugelassen ist oder nicht. Hierzu werden gebrauchsrelevante Daten aus dem elektronischen Speichermodul 20 ausgewertet, die Informationen über den bereits erfolgten oder noch nicht erfolgten Gebrauch des Einweg-Verbrauchsmaterials enthalten.

Es kann vorgesehen sein, in Abhängigkeit von den ausgelesenen gebrauchsrelevanten Daten des Speichermoduls 20 nach dem Einsetzten des Einweg-Verbrauchsmaterials Betriebsparameter beim Verwenden des Einweg-Verbrauchsmaterials einzustellen, zum Beispiel eine maximale Temperaturbelastung. Auch können ableitend von den ausgelesenen gebrauchsrelevanten Daten bei der Verwendung des Einweg-Verbrauchsmaterials im Rahmen der Bestimmung der zu untersuchenden Flüssigkeit zum Beispiel Betriebsparameter für hierbei genutzte Referenzmaterialien eingestellt werden.

Wenn nach dem Einsetzen in das Analysegerät 100 ein bestimmungsgemäßer Gebrauch des Einweg-Verbrauchsmaterials erfolgt, werden anschließend elektronische Daten betreffend diesen bestimmungsgemäßen Gebrauch in das elektronische Speichermodul 20 geschrieben. Um derartige elektronische Daten zu erzeugen, verfügt das Analysegerät 100 über geeignete Überwachungsmittel zur Erfassung von gebrauchsrelevanten Informationen, zum Beispiel in Form eines Temperatursensors, einer Uhr und / oder einer Zähleinheit.

Mit Hilfe des Temperatursensors kann beispielsweise eine Umgebungstemperatur beim Betrieb des Analysegerätes 100 erfasst werden, so dass dokumentiert ist, welchen Temperaturen das jeweilige Einweg-Verbrauchsmaterial bei seiner Nutzung ausgesetzt ist. In diesem Falle kann als gebrauchsrelevante Information beispielsweise eine kontinuierlich oder in Intervallen detektierte Temperaturkurve erfasst werden. Aus dieser können dann beispielsweise mittels geeigneter Auswerteverfahren gebrauchsrelevante Daten abgeleitet werden, beispielsweise eine kumulierte oder gemittelte Temperaturbelastung oder die Anzahl und / oder die Dauer des Über- oder Unterschreitens bestimmter vorbestimmter Temperaturgrenzen.

Mit Hilfe einer Uhr können beispielsweise Einsatz- und Entnahmepunkte eines Einweg-Verbrauchsmaterials in das Analysegerät 100 (im Sinne einer punktuellen Zeitmessung) oder auch die Verweildauer des Einweg-Verbrauchsmaterials im Analysegerät 100 (im Sinne einer Stoppuhrmessung) als gebrauchsrelevante Informationen erfasst werden, aus welchen durch geeignete Auswerteverfahren gebrauchsrelevante Daten abgeleitet werden können, beispielsweise aus punktuellen Zeitmessungen die Einsatzdauer im Analysegerät oder alternativ auch oder die Dauer des Aufenthalts des Einweg-Verbrauchsmaterials außerhalb eines Analysegeräts nach Entnahme aus dem ersten Analysegerät.

Analog können auch während des Betriebs und des Gebrauchs weitere gebrauchsrelevante Informationen mittels einer Uhr erfasst werden, beispielsweise Start und Ende von unter Einbeziehung des Einweg-Verbrauchsmaterials durchgeführten Aktionen wie Messzyklen, Kalibrierungszyklen, Qualitätskontrollzyklen, Reinigungszyklen und / oder Standby-Phasen oder deren Dauer. Hieraus können mit Hilfe geeigneter Auswerteverfahren weitere gebrauchsrelevante Daten abgeleitet werden, beispielsweise eine kumulierte Dauer, welche ein Sensor bestimmten Funktionsflüssigkeiten, zum Beispiel Reinigungs- oder Kalibrierungslösungen, ausgesetzt war, was Auswirkungen auf den weiteren Gebrauch haben kann, insbesondere auf die maximale Einsatzdauer des Sensors.

Mit Hilfe einer Zähleinheit können beispielsweise Anzahl der unter Einbeziehung des Einweg-Verbrauchsmaterials im Analysegerät bereits erfolgten oder durch rückwärtsgerichtetes Zählen Anzahl der noch möglichen Aktionen wie Messzyklen, Kalibrierungszyklen, Qualitätskontrollzyklen, Reinigungszyklen und / oder Standby-Phasen als weitere gebrauchsrelevante Informationen erfasst werden, aus welchen durch geeignete Auswerteverfahren weitere gebrauchsrelevante Daten abgeleitet werden können, beispielsweise bei Kenntnis der Dauer einer solchen Aktion/Zyklus Zeitdaten, beispielsweise eine kumulierte Dauer, welche ein bestimmter Sensor bestimmten Funktionsflüssigkeiten wie Reinigungs- oder Kalibrierungslösungen ausgesetzt war, was Auswirkungen auf den weiteren Gebrauch, insbesondere auf die maximale Einsatzdauer des Sensors haben kann.

Wenn das Einweg-Verbrauchsmaterial nach dieser Verwendung aus dem Analysegerät 100 herausgenommen wird, stehen bei einem Wiedereinsetzen in das gleiche oder ein anderes Analysegerät diese im elektronischen Speichermodul 20 gespeicherten gebrauchsrelevanten Daten diesem gleichem oder auch anderen Analysegerät für eine Auswertung zur Verfügung. Auf diese Weise können die Gebrauchsbedingungen über den Lebenszyklus des Einweg-Verbrauchsmaterials erfasst und dokumentiert werden. Insbesondere kann auf diese Weise verhindert werden, dass ein Einweg-Verbrauchsmaterial noch genutzt wird, auch wenn bei einem vorherigen Gebrauch Gebrauchsbedingungen aufgetreten sind, die wahrscheinlich oder mit Sicherheit zur Beschädigung des Einweg-Verbrauchsmaterials geführt haben. Hierüber geben die in dem elektronischen Speichermodul 20 gespeicherten gebrauchsrelevanten Daten Auskunft.

## Patentansprüche

1. Verfahren zum Überwachen der Nutzung eines Verbrauchsmaterials in Einwegausführung in mehreren Analysengeräten (100), die jeweils konfiguriert sind, eine oder mehrere Flüssigkeiten, vorzugsweise Körperflüssigkeiten, zu analysieren, wobei das Verfahren die folgenden Schritte umfasst:
- Bereitstellen eines Einweg-Verbrauchsmaterials (101, 102, 103, 104) in einem ersten Analysegerät,
- bestimmungsgemäßes Verwenden des Einweg-Verbrauchsmaterials beim Betrieb des ersten Analysegerätes,
- Erfassen von gebrauchsrelevanten Informationen für das Einweg-Verbrauchsmaterial, die das bestimmungsgemäße Verwenden des Einweg-Verbrauchsmaterials beim Betrieb des ersten Analysegerätes betreffen,
- Speichern von gebrauchsrelevanten Daten, die aus den gebrauchsrelevanten Informationen abgeleitet werden, in einer dem Einweg-Verbrauchsmaterial zugeordneten Speichereinheit (20),
- Entfernen des Einweg-Verbrauchsmaterials aus dem ersten Analysegerät,
- Bereitstellen des Einweg-Verbrauchsmaterials in einem zweiten Analysegerät,
- wenigstens teilweises Auslesen der gebrauchsrelevanten Daten aus der Speichereinheit in dem zweiten Analysegerät,
- Auswerten der ausgelesenen gebrauchsrelevanten Daten mittels einer Steuereinrichtung und
- Ableiten von Steuersignalen betreffend die Zulässigkeit und / oder die Art eines weiteren bestimmungsgemäßen Verwendens des Einweg-Verbrauchsmaterials beim Betrieb des zweiten Analysegerätes als Reaktion auf das Auswerten der ausgelesenen gebrauchsrelevanten Daten.

2. Verfahren nach Anspruch 1, **dadurch gekennzeichnet, dass** weiterhin die folgenden Schritte vorgesehen sind, wenn die Steuersignale die Zulässigkeit des weiteren bestimmungsgemäßen Verwendens des Einweg-Verbrauchsmaterials anzeigen:
- weiteres bestimmungsgemäßes Verwenden des Einweg-Verbrauchsmaterials beim Betrieb des zweiten Analysegerätes,
- Erfassen von weiteren gebrauchsrelevanten Informationen, die das weitere bestimmungsgemäße Verwenden des Einweg-Verbrauchsmaterials beim Betrieb des zweiten Analysegerätes betreffen, und
- Speichern von weiteren gebrauchsrelevanten Daten, die aus den weiteren verbrauchsmaterialrelevanten Informationen abgeleitet werden, in der dem Einweg-Verbrauchsmaterial zugeordneten Speichereinheit.

3. Verfahren nach Anspruch 1 oder 2, **dadurch gekennzeichnet, dass** aus den ausgelesenen gebrauchsrelevanten Daten die Art des weiteren bestimmungsgemäßen Verwendens des Einweg-Verbrauchsmaterials beeinflussende Daten abgeleitet werden.

4. Verfahren nach Anspruch 1, **dadurch gekennzeichnet, dass** eine auf die Unzulässigkeit des weiteren bestimmungsgemäßen Verwendens des Einweg-Verbrauchsmaterials hinweisende Benutzerinformation erzeugt wird, wenn die Steuersignale die Unzulässigkeit des weiteren bestimmungsgemäßen Verwendens des Einweg-Verbrauchsmaterials anzeigen.

5. Verfahren nach Anspruch 1 oder 4, **dadurch gekennzeichnet, dass** das weitere bestimmungsgemäße Verwenden des Einweg-Verbrauchsmaterials in dem zweiten Analysegerät unterbunden wird, wenn die Steuersignale die Unzulässigkeit des weiteren bestimmungsgemäßen Verwendens des Einweg-Verbrauchsmaterials anzeigen.

6. Verfahren nach mindestens einem der vorangehenden Ansprüche, **dadurch gekennzeichnet, dass** die erfassten gebrauchsrelevanten Informationen und die erfassten weiteren gebrauchsrelevanten Informationen ein oder mehrere Informationsaspekte ausgewählt aus der folgenden Gruppe von Informationsaspekten umfassen:
- ein oder mehrere Nutzungsbedingungen beim bestimmungsgemäßen und / oder beim weiteren bestimmungsgemäßen Verwenden des Einweg-Verbrauchsmaterials wie chemische Nutzungsparameter und / oder physikalische Nutzungsparameter,
- eine Nutzungsdauer beim bestimmungsgemäßen und / oder beim weiteren bestimmungsgemäßen Verwenden des Einweg-Verbrauchsmaterials,
- eine Nichtnutzungsdauer des Einweg-Verbrauchsmaterials nach Entnahme aus dem ersten Analysegerät,
- einen Einsatzzeitpunkt des Einweg-Verbrauchsmaterials in das oder das zweite Analysegerät,
- unter Einbeziehung des Einweg-Verbrauchsmaterials durchgeführte Aktionen wie Messzyklen, Kalibrierungszyklen, Qualitätskontrollzyklen, Reinigungszyklen und / oder Standby-Phasen und
- Verbrauchsmenge und / oder Verbrauchsumfang des Einweg-Verbrauchsmaterials beim bestimmungsgemäßen und / oder beim weiteren bestimmungsgemäßen Verwenden.

7. Verfahren nach mindestens einem der vorangehenden Ansprüche, **dadurch gekennzeichnet, dass** beim Erfassen von gebrauchsrelevanten und / oder beim Erfassen von weiteren gebrauchsrelevanten Informationen Teilinformationen in zeitlichen Abständen erfasst werden.

8. Verfahren nach mindestens einem der vorangehenden Ansprüche, dadurch **gekennzeichne**t, dass das Erfassen von gebrauchsrelevanten Informationen und / oder das Erfassen von weiteren gebrauchsrelevanten Informationen jeweils vor, während und / oder nach dem bestimmungsgemäßen / dem weiteren bestimmungsgemäßen Verwenden des Einweg-Verbrauchsmaterials erfolgt.

9. Verfahren nach mindestens einem der vorangehenden Ansprüche, dadurch **gekenn- zeichnet**, dass beim Speichern von gebrauchsrelevanten Daten und / oder Speichern von weiteren gebrauchsrelevanten Daten Teildaten in zeitlichen Abständen gespeichert werden.

10. Verfahren nach mindestens einem der vorangehenden Ansprüche, **dadurch gekennzeichnet, dass** die gebrauchsrelevanten Daten vor dem Entfernen des Einweg-Verbrauchsmaterials aus dem ersten Analysegerät und / oder die weiteren gebrauchsrelevanten Daten vor dem Entfernen des Einweg-Verbrauchsmaterials aus dem zweiten Analysegerät in der zugeordneten Speichereinheit gespeichert werden.

11. Verfahren nach mindestens einem der vorangehenden Ansprüche, **dadurch gekennzeichnet, dass** nach dem Bereitstellen des Einweg-Verbrauchsmaterials in dem ersten oder dem zweiten Analysegerät weiterhin vom Ge- oder Verbrauch des Einweg-Verbrauchsmaterials unabhängige Daten ausgelesen werden.

12. Verfahren nach mindestens einem der vorangehenden Ansprüche, dadurch **gekenn**- **zeichnet**, dass Erfassen der gebrauchsrelevanten Informationen und / oder der weiteren gebrauchsrelevanten Informationen für das Einweg-Verbrauchsmaterial mit Hilfe einer Überwachungseinrichtung des ersten / zweiten Analysegerätes ausgeführt wird.

13. Verfahren nach mindestens einem der vorangehenden Ansprüche, **dadurch gekennzeichnet, dass** Speichern der gebrauchsrelevanten Daten und / oder das Speichern der weiteren gebrauchsrelevanten Daten in der dem Einweg-Verbrauchsmaterial zugeordneten Speichereinheit mit Hilfe einer Schreibeinrichtung des ersten / zweiten Analysegerätes ausgeführt wird.

14. Verfahren nach mindestens einem der vorangehenden Ansprüche, dadurch **gekenn**- **zeichnet**, dass wenigstens teilweises Auslesen der gebrauchsrelevanten Daten aus der Speichereinheit mit Hilfe einer Leseeinrichtung des ersten / zweiten Analysegerätes ausgeführt wird.

## Claims

1. Method for monitoring the use of a consumable in a disposable design in several analyzers (100) which are each configured to analyse one or more liquids, preferably body fluids, wherein the method comprises the following steps:
- providing a disposable consumable (101, 102, 103, 104) in a first analyzer,
- use of the disposable consumable as intended during operation of the first analyzer,
- collecting usage-relevant information for the disposable consumable which concern the intended use of the disposable consumable during operation of the first analyzer,
- storing usage-relevant data which are derived from the usage-relevant information in a memory unit (20) assigned to the disposable consumable,
- removing the disposable consumable from the first analyzer,
- providing the disposable consumable in a second analyzer,
- at least partially reading the usage-relevant data from the memory unit in the second analyzer,
- evaluating the usage-relevant data that have been read out by means of a control device, and
- deriving control signals concerning the permissibility and/or type of a further intended use of the disposable consumable during operation of the second analyzer as a reaction to the evaluation of the usage-relevant data that have been read out.

2. Method according to claim 1, **characterized in that** the following steps are additionally provided when the control signals indicate the permissibility of the continued intended use of the disposable consumable:
- continued intended use of the disposable consumable during the operation of the second analyzer,
- collection of further usage-relevant information which concern the further intended use of the disposable consumable during the operation of the second analyzer, and
- storage of further usage-relevant data which are derived from the further consumable-relevant information in the memory unit assigned to the disposable consumable.

3. Method according to claim 1 or 2, **characterized in that** data are derived from the usage-relevant data that are read out which influence the manner in which the disposable consumable is subsequently used as intended.

4. Method according to claim 1, **characterized in that** a user information indicating the inadmissibility of the further intended use of the disposable consumable is generated when the control signals indicate the inadmissibility of the further intended use of the disposable consumable.

5. Method according to claim 1 or 4, **characterized in that** further intended use of the disposable consumable in the second analyzer is prevented when the control signals indicate the inadmissibility of the further intended use of the disposable consumable.

6. Method according to at least one of the previous claims, **characterized in that** the collected usage-relevant information and the collected further usage-relevant information comprises one or more information aspects selected from the following group of information aspects:
- one or more conditions of use for the intended use or further intended use of the disposable consumable such as chemical usage parameters and/or physical usage parameters,
- a period of use for the intended use and/or for the further intended use of the disposable consumable,
- a period of non-use of the disposable consumable after it has been removed from the first analyzer,
- a time at which the disposable consumable is inserted into the second analyzer,
- the actions carried out with the aid of the disposable consumable such as measuring cycles, calibration cycles, quality control cycles, cleaning cycles and/or standby phases, and
- the amount consumed and/or extent of consumption of the disposable consumable during its intended use and/or during its further intended use.

7. Method according to at least one of the previous claims, **characterized in that** when collecting usage-relevant information and/or when collecting further usage-relevant information, partial information is collected at time intervals.

8. Method according to at least one of the previous claims, **characterized in that** the collection of usage-relevant information and/or the collection of further usage-relevant information takes place in each case before, during and/or after the intended use or the further intended use of the disposable consumable.

9. Method according to at least one of the previous claims, **characterized in that** partial data are stored at intervals when storing usage-relevant data and/or storing further usage-relevant data.

10. Method according to at least one of the previous claims, **characterized in that** the usage-relevant data are stored in the assigned memory unit before the disposable consumable is removed from the first analyzer and/or the further usage-relevant data are stored in the assigned memory unit before the disposable consumable is removed from the second analyzer.

11. Method according to at least one of the previous claims, **characterized in that** after the disposable consumable has been provided in the first or the second analyzer, data that are independent of the usage or consumption of the disposable consumable continue to be read out.

12. Method according to at least one of the previous claims, **characterized in that** the usage-relevant information and/or the further usage-relevant information for the disposable consumable is collected with the aid of a monitoring device of the first / second analyzer.

13. Method according to at least one of the previous claims, **characterized in that** the storage of usage-relevant data and/or the storage of further usage-relevant data in the memory unit assigned to the disposable consumable is stored with the aid of a writing device of the first / second analyzer.

14. Method according to at least one of the previous claims, **characterized in that** the usage-relevant data are at least partially read from the memory unit with the aid of a reading device of the first / second analyzer.

## Revendications

1. Procédé de surveillance de l'utilisation d'une substance consommable en modèle à usage unique dans plusieurs appareils d'analyse (100) qui sont respectivement configurés pour analyser un ou plusieurs liquides, de préférence des liquides physiologiques, ce procédé comprenant les étapes suivantes consistant à :
- mettre une substance consommable à usage unique (101, 102, 103, 104) dans un appareil d'analyse,
- utiliser la substance consommable à usage unique conformément à sa destination pendant le fonctionnement du premier appareil d'analyse,
- enregistrer pour la substance consommable à usage unique des informations concernant la consommation qui se rapportent à l'utilisation conforme à sa destination de la substance consommable à usage unique pendant le fonctionnement du premier appareil d'analyse,
- enregistrer des données concernant la consommation qui sont dérivées des informations concernant la consommation dans une unité de sauvegarde (20) associée à la substance consommable à usage unique,
- retirer la substance consommable à usage unique du premier appareil d'analyse,
- mettre la substance consommable à usage unique dans un second appareil d'analyse,
- lire au moins partiellement les données concernant la consommation dans l'unité de sauvegarde du second appareil d'analyse,
- exploiter les données concernant la consommation lues au moyen d'une unité de commande et
- dériver des signaux de commande concernant l'admissibilité et/ou le type d'utilisation conforme à la destination de la substance consommable à usage unique pendant le fonctionnement du second appareil d'analyse en réaction à l'exploitation des données concernant la consommation lues.

2. Procédé selon la revendication 1, **caractérisé en ce que** sont prévues en outre les étapes suivantes si les signaux de commande indiquent l'admissibilité de la poursuite de l'utilisation conforme à la destination de la substance consommable à usage unique, ces étapes consistant à :
- poursuivre l'utilisation conforme à la destination de la substance consommable à usage unique pendant le fonctionnement du second appareil d'analyse,
- enregistrer pour la substance consommable à usage unique d'autres informations concernant la consommation qui se rapportent à l'utilisation conforme à sa destination de la substance consommable à usage unique pendant le fonctionnement du second appareil d'analyse,
- enregistrer d'autres données concernant la consommation qui sont dérivées des autres informations concernant la consommation de substance dans une unité de sauvegarde associée à la substance consommable à usage unique.

3. Procédé selon la revendication 1 ou 2, **caractérisé en ce que** des données jouant sur le type de poursuite de l'utilisation conforme à la destination de la substance consommable à usage unique sont dérivées des données concernant la consommation lues.

4. Procédé selon la revendication 1, **caractérisé en ce qu'**est générée une information destinée à l'utilisateur signalant l'inadmissibilité de la poursuite de l'utilisation conforme à la destination de la substance consommable à usage unique si les signaux de commande indiquent l'inadmissibilité de la poursuite de l'utilisation conforme à la destination de la substance consommable à usage unique.

5. Procédé selon la revendication 1 ou 4, **caractérisé en ce que** la poursuite de l'utilisation conforme à la destination de la substance consommable à usage unique dans le second appareil d'analyse est bloquée si les signaux de commande indiquent l'inadmissibilité de la poursuite de l'utilisation conforme à la destination de la substance consommable à usage unique.

6. Procédé selon au moins une des revendications précédentes, **caractérisé en ce que** les données concernant la consommation enregistrées et les autres informations concernant la consommation enregistrées comprennent ou plusieurs aspects d'information sélectionnés dans le groupe suivant d'aspects d'information :
- une ou plusieurs conditions d'utilisation en cas d'utilisation conforme à la destination et/ou en cas de poursuite d'utilisation conforme à la destination de la substance consommable à usage unique, comme des paramètres d'utilisation chimiques et/ou des paramètres d'utilisation physiques,
- une durée d'utilisation en cas d'utilisation conforme à la destination et/ou en cas de poursuite de l'utilisation conforme à la destination de la substance consommable à usage unique,
- une durée de non-utilisation de la substance consommable à usage unique après son retrait du premier appareil d'analyse,
- un moment d'incorporation de la substance consommable à usage unique dans l'appareil ou le second appareil d'analyse,
- des actions réalisées en intégrant la substance consommable à usage unique comme des cycles de mesure, des cycles d'étalonnage, des cycles de contrôle de qualité, des cycles de nettoyage et/ou des phases de veille et
- une quantité de consommation et/ou un volume de consommation de la substance consommable à usage unique en cas d'utilisation conforme à la destination et/ou en cas de poursuite d'utilisation conforme à la destination.

7. Procédé selon au moins une des revendications précédentes, **caractérisé en ce que** des informations partielles sont enregistrées périodiquement lors de l'enregistrement de données concernant la consommation et/ou lors de l'enregistrement d'autres informations concernant la consommation.

8. Procédé selon au moins une des revendications précédentes, **caractérisé en ce que** l'enregistrement d'informations concernant la consommation et/ou l'enregistrement d'autres informations concernant la consommation a lieu respectivement avant, pendant et/ou après l'utilisation conforme à la destination/la poursuite de l'utilisation conforme à la destination de la substance consommable à usage unique.

9. Procédé selon au moins une des revendications précédentes, **caractérisé en ce que** lors de l'enregistrement d'informations concernant la consommation et/ou de l'enregistrement d'autres informations concernant la consommation, des données partielles sont enregistrées périodiquement.

10. Procédé selon au moins une des revendications précédentes, **caractérisé en ce que** les données concernant la consommation sont enregistrées avant le retrait de la substance consommable à usage unique du premier appareil d'analyse et/ou les autres données concernant la consommation avant le retrait de la substance consommable à usage unique du second appareil d'analyse dans l'unité de sauvegarde associée.

11. Procédé selon au moins une des revendications précédentes, **caractérisé en ce que**, après avoir mis la substance consommable à usage unique dans le premier ou le second appareil d'analyse, des données indépendantes de l'usage ou de la consommation de de la substance consommable à usage unique sont lues.

12. Procédé selon au moins une des revendications précédentes, **caractérisé en ce que** l'enregistrement des données concernant la consommation et/ou des autres données concernant la consommation de la substance consommable à usage unique est réalisé à l'aide d'un dispositif de surveillance du premier/second appareil d'analyse.

13. Procédé selon au moins une des revendications précédentes, **caractérisé en ce que** l'enregistrement des données concernant la consommation et/ou l'enregistrement des autres données concernant la consommation est réalisé dans une unité de sauvegarde associée à la substance consommable à usage unique à l'aide d'un dispositif d'écriture du premier/second appareil d'analyse.

14. Procédé selon au moins une des revendications précédentes, **caractérisé en ce que** la lecture au moins partielle des données concernant la consommation dans l'unité de sauvegarde se fait à l'aide d'un dispositif de lecture du premier/second appareil d'analyse.
